# EUROPEAN PATENT APPLICATION

(11) **EP 3 178 442 A1**
(43) Date of publication of application: **14.06.2017**
(21) Application number: 17151075.3
(22) Date of filing: 05.12.2008
(51) Int. Cl.: A61F 2/00

(54) **IMPLANTABLE PROSTHESIS**

(30) Priority: 07.12.2007 US 5738 P
(62) Divisional of application: 08859873.5
(71) Applicant: C.R. Bard Inc., Murray Hill, NJ 07974 (US)
(72) Inventor: KULLAS,, Karen E., Berkley, Massachusetts 02779-1115 (US)
(74) Representative: Grey, Ian Michael

(57) **Abstract**

A prosthetic device (10) for repairing or augmenting a tissue, muscle or organ defect is disclosed. It comprises a first portion (12) of biological tissue that is remodelable and configured for tissue ingrowth. The first portion being constructed and arranged to cover or plug a defect; and a second portion (14) of biological tissue that is more slowly enzymatically degradable after implantation than the first layer and that is not configured for tissue ingrowth. The second portion has sufficient pullout strength so that said prosthetic device is fixatable through said second portion.

## Description

### FIELD OF THE INVENTION

The present invention relates to prosthetic devices, and methods of using such devices for repairing, reconstructing, buttressing, or augmenting a defect or weakness in a wall of a tissue, muscle or organ.

### BACKGROUND OF THE INVENTION

Various prosthetic repair materials have been proposed to reinforce the abdominal wall and to close abdominal wall defects. One common approach to repair or reinforce a weakened abdominal wall employs a porous, knitted, synthetic mesh implant. Such a flexible and porous implant provides strength for reinforcing tissue and closing tissue defects, particularly in the abdominal cavity, while preserving sufficient openings in the material to support native tissue ingrowth. In certain procedures, however, the prosthetic mesh may come into direct contact with the sensitive abdominal viscera, potentially leading to postoperative adhesions between the mesh and, for example, abdominal contents, i.e., mesentery, bowel and intestines.

Various approaches to reducing the incidence of postoperative adhesions arising from the use of prosthetic mesh materials have been proposed by the prior art. One known approach employs a composite prosthesis, which combines a porous knitted mesh layer with a barrier material. Typically, the porous layer supports tissue ingrowth and provides mechanical strength as it bridges the defect while the barrier layer, which may principally be non-load bearing, reduces the potential for intestinal adhesions.

The earliest composite prostheses were entirely synthetic, resulting in a permanent implant. More recently, efforts to reduce the presence of foreign matter in the body have led to the development of at least partially biologic implants, in which biologic components are gradually resorbed by the body. One such biologic implant is the COLLAMEND [TM] implant available from C.R.Bard, Inc. of Murray Hill, New Jersey. The COLLAMEND [TM] material, formed of a single layer of acellular, lyophilized, crosslinked porcine dermis, is a fully resorbable implant exhibiting the required strength and durability for soft tissue reinforcement and tissue defect repair.

### SUMMARY OF THE INVENTION

According to an aspect of the invention, there is provided a prosthetic device for repairing or augmenting a tissue, muscle or organ defect, comprising: a first portion of biological tissue that is remodelable and configured for tissue ingrowth, said first portion constructed and arranged to cover or plug a defect; and a second portion of biological tissue that is more slowly enzymatically degradable after implantation than the first layer and that is not configured for tissue ingrowth, said second portion having sufficient pullout strength so that said prosthetic device is fixatable through said second portion.

Preferred features of the prosthetic device according to the invention are defined in the dependent claims.

Another aspect of the invention includes a prosthetic device for repairing or augmenting a tissue, muscle or organ defect, the prosthetic device including a first portion of biological tissue that is rapidly enzymatically degradable after implantation, remodelable, and configured for tissue ingrowth, with the first portion constructed and arranged to cover or plug a defect. The prosthetic device also includes a second portion of biological tissue that is slowly enzymatically degradable after implantation and that is not configured for tissue ingrowth, the second portion having sufficient pullout strength so that the prosthetic device is fixatable through the second portion.

The first portion of biological tissue may not be cross-linked or may be minimally cross-linked.

The second portion of biological tissue may be cross-linked.

The second portion of biological tissue may include biological tissue that has been processed with resulting increased pullout strength.

At least 50% of said first portion of biological tissue may be resorbable in less than twelve weeks after implantation.

At least 50% of said second portion of biological tissue may be resorbable in no less than fifty-two weeks.

The first portion of biological tissue may have a thickness of between about 0.3 mm and 0.5 mm.

The second portion of biological tissue may have a thickness of between about 0.7 mm and 1.4 mm.

The first and second portions of biological tissue may be acellular, lyophilized, porcine dermis.

The first portion of biological tissue may have a first surface and a second surface and includes holes extending from said first surface to said second surface that are configured for tissue ingrowth.

The first portion of biological tissue may include a surface that is textured for tissue ingrowth.

The first and second portions of biological tissue may be in the form of a patch.

The patch may include a sheet of said second portion of biological tissue that surrounds at least an aspect of a sheet of said first portion of biological tissue.

The sheet of said first portion of biological tissue may be positioned against said sheet of said second portion of biological tissue.

The sheet of said first portion of biological tissue may be positioned either face-to-face, or edge-to-edge, with said sheet of said second portion of biological tissue.

The sheet of said first portion of biological tissue may be attached to said sheet of said second portion of biological tissue by a resorbable material.

The second portion of biological tissue may have a ball burst strength at least about twice that of said first portion of biological tissue.

The second portion of biological tissue may have a ball burst strength of at least about three times that of said first portion of biological tissue.

The second portion of biological tissue may have a ball burst strength of at least about four times that of said first portion of biological tissue.

The prosthetic device may be in the form of a plug.

The first portion of biological tissue may be in the form of a plug and said second portion of biological tissue may be in the form of a patch.

Another aspect of the invention includes a prosthetic device for repairing or augmenting a tissue, muscle or organ defect, the prosthetic device including a non-crosslinked, or minimally cross-linked, portion of biological tissue that is constructed and arranged to cover or plug a defect. The prosthetic device also includes a cross-linked portion of biological tissue having sufficient pullout strength so that the prosthetic device is fixatable through the second portion.

At least 50% of said non-crosslinked, or minimally cross-linked, portion of biological tissue may be resorbable in less than twelve weeks after implantation.

At least 50% of said cross-linked portion of biological tissue may be resorbable in no less than fifty-two weeks after implantation.

The non-crosslinked, or minimally cross-linked, portion of biological tissue may have a thickness of between about 0.3 mm and 0.5 mm.

The cross-linked portion of biological tissue may have a thickness of between about 0.7 mm and 1.4 mm.

The non-crosslinked, or minimally cross-linked, portion of biological tissue and said cross-linked portion of biological tissue may be acellular, lyophilized, porcine dermis.

The non-crosslinked, or minimally cross-linked, portion of biological tissue may have a first surface and a second surface and may include holes extending from said first surface to said second surface that are configured for tissue ingrowth.

The non-crosslinked, or minimally cross-linked, portion of biological tissue may include a surface that is textured for tissue ingrowth.

The non-crosslinked, or minimally cross-linked, portion of biological tissue and said cross-linked portion of biological tissue may be in the form of a patch.

The patch may include a sheet of said cross-linked portion of biological tissue that surrounds at least an aspect of a sheet of said non-crosslinked, or minimally cross-linked, portion of biological tissue.

The sheet of non-crosslinked, or minimally cross-linked, portion of biological tissue may be positioned against said sheet of cross-linked portion of biological tissue.

The sheet of non-crosslinked, or minimally cross-linked, portion of biological tissue may be positioned either face-to-face, or edge-to-edge, with said sheet of cross-linked portion of biological tissue.

The sheet of non-crosslinked, or minimally cross-linked, portion of biological tissue may be attached to said sheet of cross-linked portion of biological tissue by a resorbable material.

The cross-linked portion of biological tissue may have a ball burst strength at least about twice that of said non-crosslinked, or minimally cross-linked, portion of biological tissue.

The cross-linked portion of biological tissue may have a ball burst strength of at least about three times that of said non-crosslinked, or minimally cross-linked, portion of biological tissue.

The cross-linked portion of biological tissue may have a ball burst strength of at least about four times that of said non-crosslinked, or minimally cross-linked, portion of biological tissue.

The prosthetic device may be in the form of a plug.

The non-crosslinked, or minimally cross-linked, portion of biological tissue may be in the form of a plug and said cross-linked portion of biological tissue may be in the form of a patch.

Another aspect of the present invention includes a prosthetic device for repairing or augmenting a tissue, muscle or organ defect, the prosthetic device including a first portion of acellular, lyophilized, porcine dermis, at least 50% of the first portion being resorbable in less than twelve weeks after implantation, the first portion being configured for tissue ingrowth. The prosthetic device also includes a second portion of acellular, lyophilized, porcine dermis, at least 50% of the second portion being resorbable in no less than fifty-two weeks after implantation, the second portion not being configured for tissue ingrowth, wherein the second portion is arranged to provide an accessible fixation location for the prosthetic device.

The first portion may have a thickness of between about 0.3 mm and 0.5 mm.

The second portion may have a thickness of between about 0.7 mm and 1.4 mm.

The first portion may have a first surface and a second surface and includes holes extending from said first surface to said second surface that are configured for tissue ingrowth.

The first portion may include a surface that is textured for tissue ingrowth.

The first portion and said second portion may be in the form of a patch.

The patch may include a sheet of said second portion that surrounds at least an aspect of a sheet of said first portion.

The sheet of said first portion may be positioned against said sheet of said second portion.

The sheet of said first portion may be positioned either face-to-face, or edge-to-edge, with said sheet of said second portion.

The sheet of said first portion may be attached to said sheet of said second portion by a resorbable material.

The second portion may have a ball burst strength at least about twice that of said first portion.

The second portion may have a ball burst strength of at least about three times that of said first portion.

The second portion may have a ball burst strength of at least about four times that of said first portion.

The prosthetic device may be in the form of a plug.

The first portion may be in the form of a plug and said second portion may be in the form of a patch.

Another aspect of the present invention includes a prosthetic device for repairing or augmenting a tissue, muscle or organ defect, the prosthetic device having a first portion of biological tissue that is configured for tissue ingrowth, the first portion having a ball burst strength. The prosthetic device also includes a second portion of biological tissue, the second portion having a ball burst strength greater than the first portion ball burst strength, wherein the second portion surrounds at least an aspect of the first portion.

The first portion of biological tissue may not be cross-linked or may be minimally cross-linked.

The second portion of biological tissue may be cross-linked.

The second portion of biological tissue may include biological tissue that has been processed with resulting increased ball burst strength.

At least 50% of said first portion of biological tissue may be resorbable in less than twelve weeks after implantation.

At least 50% of said second portion of biological tissue may be resorbable in no less than fifty-two weeks.

The first portion of biological tissue may have a thickness of between about 0.3 mm and 0.5 mm.

The second portion of biological tissue may have a thickness of between about 0.7 mm and 1.4 mm.

The first and second portions of biological tissue may be are acellular, lyophilized, porcine dermis.

The first portion of biological tissue may have a first surface and a second surface and may include holes extending from said first surface to said second surface that are configured for tissue ingrowth.

The first portion of biological tissue may include a surface that is textured for tissue ingrowth.

The first and second portions of biological tissue may be in the form of a patch.

The patch may include a sheet of said second portion of biological tissue that surrounds at least an aspect of a sheet of said first portion of biological tissue.

The sheet of said first portion of biological tissue may be positioned against said sheet of said second portion of biological tissue.

The sheet of said first portion of biological tissue may be positioned either face-to-face, or edge-to-edge, with said sheet of said second portion of biological tissue.

The sheet of said first portion of biological tissue may be attached to said sheet of said second portion of biological tissue by a resorbable material.

The second portion of biological tissue may have a ball burst strength at least about twice that of said first portion of biological tissue.

The second portion of biological tissue may have a ball burst strength of at least about three times that of said first portion of biological tissue.

The second portion of biological tissue may have a ball burst strength of at least about four times that of said first portion of biological tissue.

The prosthetic device may be in the form of a plug.

The first portion of biological tissue may be in the form of a plug and said second portion of biological tissue may be in the form of a patch.

Another aspect of the present invention includes a method of repairing or augmenting a tissue, muscle or organ defect with a prosthetic device, the prosthetic device having a first portion of biological tissue that is rapidly enzymatically degradable after implantation and that is configured for tissue ingrowth and a second portion of biological tissue that is slowly enzymatically degradable after implantation and that is not configured for tissue ingrowth. The method involves positioning the prosthetic device so that the first portion covers or plugs a defect and fixating the second portion to secure the first portion in place relative to the defect.

The repair or augmentation may be an intraperitoneal procedure.

The fixating may include one or more of suturing, stapling, or adhesively bonding the second portion.

The positioning step may include plugging the soft tissue defect with the first portion and covering the soft tissue defect with the second portion.

The tissue defect has an entrance and an exit, and said covering may include covering either, or both, of the entrance or exit with the second portion.

The tissue defect has an entrance and an exit, and said covering may include covering either the entrance or the exit with the first portion.

The repair or augmentation may be performed laparoscopically.

Another aspect of the present invention is a prosthetic repair patch comprising: a second layer of biologically compatible and resorbable material having a microporous structure that inhibits native tissue ingrowth; and a first layer of biologically compatible and resorbable material attached to the second layer, wherein the first and second layers have different rates of resorption and the first layer includes a plurality of openings to allow native tissue ingrowth and vascularization.

The first and second layers may be sewn together with a resorbable suture.

The second layer may have a maximum width and the first layer may have a maximum width, wherein the maximum width of the second layer may be at least about twice the maximum width of the first layer.

The second layer may have a substantially uniform thickness and the first layer may have a substantially uniform thickness, wherein the thickness of the second layer may be at least about twice the thickness of the first layer.

The second layer may have a rate of resorption which is at least about ten times longer than the rate of resorption of the first layer.

The second layer may have a rate of resorption which is at least about twenty times longer than the rate of resorption of the first layer.

The first and second layers may be made of porcine dermis.

Only one of the layers may be crosslinked.

The first layer may have a top surface and a bottom surface and may include at least one opening extending from the top surface to the bottom surface.

Another aspect of the present invention is an implantable prosthesis comprising: a second layer of crosslinked porcine dermis and a first layer of non-crosslinked porcine dermis, wherein the first layer has a plurality of openings of sufficient size to allow native tissue ingrowth.

According to another aspect of the invention, there is provided an implantable prosthesis comprising a resorbable layer having a plurality of spaced apart through cuts forming hinged tabs that are foldable out of the plane of the layer to define corresponding through holes in the layer.

The resorbable layer may be formed of porcine dermis.

The through cuts may be arcuate.

### BRIEF DESCRIPTION OF THE DRAWINGS

Other aspects, advantages and novel features will become more apparent from the following detailed description of embodiments of the invention when considered in conjunction with the accompanying drawings wherein:
FIG. 1 is an illustration of a composite two-layer resorbable prosthesis according to one embodiment of the present invention;
FIG 2. is an enlarged cross-sectional view taken along section line 2-2 of FIG. 1;
FIG. 3 is an illustration of a two-layer resorbable prosthesis according to another embodiment of the invention;
FIG. 4 is an enlarged cross-sectional view taken along section line 4-4 of FIG.3; and
FIG 5. is an illustration of the bottom side of the prosthesis shown in FIGS. 3 and 4.

### DETAILED DESCRIPTION

Aspects of the invention include a prosthetic device for repairing or augmenting a weakened or damaged tissue wall, muscle or organ (i.e., a defect), and a method of repairing or augmenting a weakened or damaged tissue wall, muscle or organ with a prosthetic device. Embodiments of the prosthesis may be particularly suited for procedures involving inguinal, femoral, hiatal, ventral, incisional and recurrent hernias, chest or abdominal wall reconstructions and augmentations, and the treatment of other large defects such as those that may occur in obese patients. The prosthetic device is not limited to a particular configuration and may, for example, be in the form of a patch, a plug, a patch and plug combination, or other arrangement. The prosthetic device may be employed in an open procedure, laparoscopic procedure, hybrid open or laparoscopic procedure, as well as other surgical methodologies and, in connection with abdominal wall procedures, may be performed preperitoneal or intraperitoneal.

In certain embodiments, the prosthetic device includes one or more portions or layers of biological tissue, such as human and/or animal tissue, that may be processed for implantation in a human or animal, with acellular, lyophilized, porcine dermis being a particularly suitable material. Some aspects, or all, of a prosthetic device may include one or more of the following features: enzymatically degradable, remodelable, crosslinked, and/or configured for tissue ingrowth. Any of the foregoing properties, if provided, may be present uniformly throughout the prosthetic device or, instead, may vary at different locations of the implant. Further, the strength of the prosthetic device may substantially differ amongst disparate regions, with such strength being characterizable, for example, by a ball burst strength. The prosthetic device may be loaded, coated, impregnated or otherwise contain an anesthetic, antibiotic, drug or other medicament.

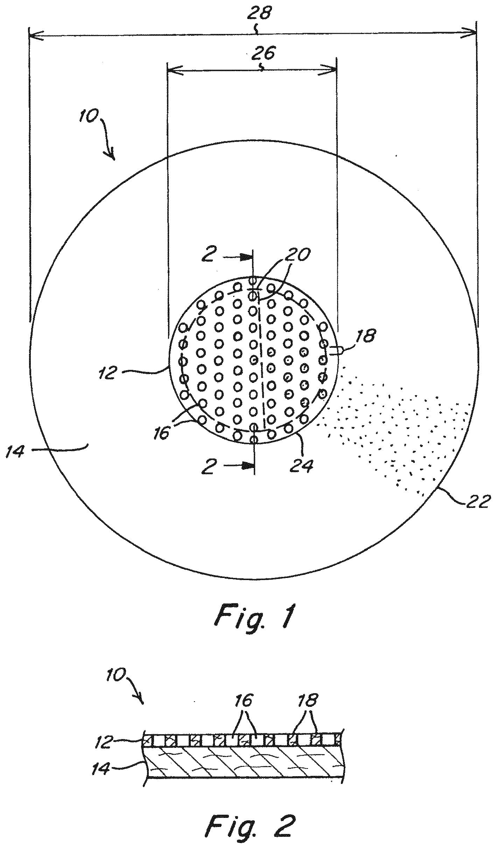

According to one embodiment of the invention, illustrated in FIGS. 1 and 2, a prosthesis 10 includes a first portion or layer 12 of biological tissue (e.g., autograft, allograft, xenograft, or combinations of any of the foregoing) that is configured to permit tissue infiltration and vascularization after implantation. The first portion or layer may be formed of acellular, lyophilized, porcine dermis having a thickness of between about 0.3 mm and 0.5 mm. The first layer or portion may be porous and/or may include a plurality of holes 16 that have been punched, die cut or otherwise formed therethrough to facilitate tissue ingrowth. The holes may have a diameter of about 0.060 to 0.094 inches and be spaced apart via a material web 18 between about 0.47 and 0.94 inches. It will be appreciated that the exact size, number, presence or absence, position and spacing of the holes may be varied to achieve the desired performance characteristics. Alternatively, or additionally, a surface of the ingrowth layer may be scraped, scored or otherwise treated to provide a surface texture that facilitates tissue ingrowth. Other biologic materials also are contemplated for the tissue ingrowth portion or layer including, for example, COLLAMEND [TM], ALLOMAX [TM], or ULTRAFOAM [TM] sponge, all of which are available from C.R. Bard, Inc. of Murray Hill, New Jersey. Synthetic resorbable, nonresorbable and partially resorbable materials also are contemplated for the ingrowth layer or portion including knitted polypropylene or polyester filaments. Certain embodiments of a tissue infiltratable layer or portion may be cross-linked; for example, porcine dermis may be crosslinked using EDAC (1-ethyl-(3-dimethylaminopropyl)-carbodiimide hydrochloride). Alternatively, a tissue infiltratable layer 12 may not be crosslinked at all, or may be minimally crosslinked.

A second portion or layer 14 of the composite prosthesis also may be formed of a biological tissue. The second portion or layer may not be configured for tissue ingrowth; that is, the biological tissue may be substantially non-porous, not include any additionally formed openings therethrough, or otherwise not be receptive to tissue infiltration initially upon implantation (however, over time as the second portion is enzymatically attacked it may, ultimately, become susceptible to tissue ingrowth). The second portion or layer may be in the form of a barrier that does not stimulate adhesion formation when implanted. In applications where the second portion or layer includes, or is in the form of, a barrier, the barrier may be positioned to isolate the tissue infiltratable first portion or layer from sensitive tissue and organs that it may come into contact with, limiting the incidence of postoperative adhesions with the prosthetic device. Although not limited to a particular dimension, in certain embodiments the second portion or layer may have a thickness ranging from about 0.7 mm to 1.4 mm.

The second portion or layer may be sufficiently strong to prevent unwanted pullout of anchoring sutures, tacks, or other fixation constructs that may be used in a surgical procedure. The second portion or layer may be substantially stronger than the tissue infiltratable portion or layer, and may be characterized by a burst strength that is at least two times, three times, or four times greater than the burst strength of the first portion or layer. The second portion or layer may be formed of acellular, lyophilized, cross-linked porcine dermis (such as COLLAMEND [TM]). This particular construction may serve as a barrier as it is not receptive to viscera adhesion. Also, the second portion or layer may include one or more through cuts or other openings to reduce the potential for fluid accumulation between the first and second layers. Other autogeneous, heterogenous and xenogenic tissues also are contemplated for the barrier layer including, to name a few, human or animal (e.g., bovine, porcine) dermis, pericardium and small intestine submucosa. The second portion or layer may be crosslinked, minimally crosslinked, or not crosslinked at all. In certain embodiments, other non-crosslinking approaches may be employed to increase the strength of the second portion or layer.

The two portions or layers of the prosthesis 10 may be placed separately at the surgical site or may be in united form prior to implantation (for example, as supplied in sterile form by a medical device company). Connection of the two portions or layers preferably is by stitching 20 using resorbable suture material, for example, PDO or polydioxanone. Other mechanisms for connecting the two portions or layers, such as tacking, stapling, gluing and chemical bonding also are within the scope of the invention so long as the rendered prosthesis provides the desired performance characteristics. In certain embodiments, the rate of resorption may vary between the tissue infiltratable layer or portion and the second layer or portion, with the tissue infiltratable layer or portion resorbing more rapidly. For example, and without limitation, at least 50% of the tissue infiltratable portion or layer may be resorbable in less than twelve weeks after implantation, while at least 50% of the second portion or layer may be resorbable in no less than fifty-two weeks. In other embodiments, the second portion or layer may have a rate of resorption at least ten times longer, or twenty times longer, than the rate of resorption of the tissue infiltratable portion or layer. Resorbability, or rate or resorption, may be assessed in various way as should be apparent to one of skill in the art. Representative approaches include assessing either changes in weight or thickness, as well as by histological observation.

As shown in the figures, the two layers or portions may be joined face-to-face. In other embodiments, the two layers or portions may be joined edge-to-edge, where the adjoining edges are external edges, or may be an external edge adjacent an internal edge (for example, where a second portion or layer circumscribes a first portion or layer). In the edge-to-edge arrangements, the adjacent edges need not be contiguous; they may overlap or an intermediate structure may be provided therebetween. Similarly, in the face-to-face embodiment, an intermediate structure may separate the tissue infiltratable layer or portion and the second layer or portion. Other juxtapositions of the two layers or portions also are contemplated.

Although shown and described in the form of a patch, the prosthetic device is not limited to a particular configuration. Other contemplated arrangements, without limitation, include a plug and a patch/plug combination. In one embodiment, the tissue infiltratable layer or portion may be in the form of a plug and the second portion or layer in the form of an underlay, overlay or both an underlay and overlay. The plug may have a cylindrical, conical, or other three-dimensional shape, while the underlay and/or overlay may have, or may be arranged to have, a substantially planar, convex, concave, or other shape. The plug may be minimally crosslinked or not crosslinked at all, while the underlay and/or overlay may be crosslinked. Such a plug and patch combination may be surgically placed so that the plug resides in the defect, and the overlay (if provided) extends across an entrance to the defect and the underlay (if provided) extends across an exit to the defect. In any of the embodiments described herein, the prosthetic device may be imparted with flexibility as desired; for example, to facilitate handling, to conform to the defect, and/or to reduce detection of the device by the patient post implantation.

In certain embodiments, the tissue infiltratable portion or layer 12 is smaller in size than the second portion or layer 14, wherein the periphery of the second portion or layer 22 extends well beyond the outmost edge 24 of the tissue infiltratable portion or layer. For example, the smaller tissue infiltratable portion or layer may have a diameter 26 of about one-third to one-half to the diameter 28 of the second portion or layer. Configured this way, the centrally or otherwise located tissue infiltratable portion or layer may be positioned over a tissue gap or hernia defect, providing a matrix for accelerated ingrowth of bridging tissue. At the same time, the second portion or layer would extend sufficiently beyond the margins of the gap, and provide an accessible fixation location to permit secure attachment to surrounding tissue using adhesives or fixation constructs. Once secured at, or over, the repair site, the second portion or layer provides mechanical integrity and strength to the repair. In certain embodiments, a stronger second portion or layer compensates for the tendency of a flimsier tissue infiltratable portion or layer to bunch up, wrinkle or curl (as may occur where the tissue infiltratable portion or layer, alone, is attached directly to tissue surrounding the defect). Over time, the infiltratable portion or layer, the second portion or layer, and the resorbable sutures or other fixation mechanism will be resorbed by the body, with only native tissue remaining as a permanent repair of the defect.

In certain embodiments, the second portion or layer has a maximum width that is at least about twice the maximum width of the tissue infiltratable portion or layer. In other embodiments, the tissue infiltratable portion or layer and second portion or layer each have a substantially uniform thickness, and the second portion or layer is at least about twice the thickness of the tissue infiltratable portion or layer.

A representative procedure for forming a dual layer prosthesis, where the tissue infiltratable portion or layer is in the form of a sheet, and the second portion or layer also is in the form of a sheet, and the two sheets are joined face-to-face, according to the present invention will now be described. A piece of COLLAMEND [TM] porcine dermis, commercially available from C.R. Bard, Inc. of Murray Hill, New Jersey is die cut to form the tissue ingrowth sheet. A steel rule die is used to cut a 1.375 inch disk of material, and a plurality of 0.094 inch diameter, spaced, holes are formed, which are spaced apart with a web of 0.47 to 0.60 inches. A second piece of COLLAMEND [TM] material is then die cut to a diameter of 3.5 inches. No holes are placed in this layer. The smaller ingrowth layer may be placed centrally on the larger layer, or offset, and the layers are stitched together using a commercially available resorbable suture, such as a PROLENE [TM] suture.

The representative embodiment of a composite implant includes a fenestrated, minimally or non-crosslinked, resorbable ingrowth layer attached to a microporous, resorbable, stronger and/or barrier layer. The fully crosslinked stronger and/or barrier layer has a diameter of 3.5 inches and a thickness of 0.039 inch. The ingrowth layer has a diameter of 1.375 inches and a thickness of 0.012 inch. It will be appreciated that the materials, degree of crosslinking, mesh diameters, thicknesses, hole size, position and spacing maybe varied in the foregoing example to suit particular needs.

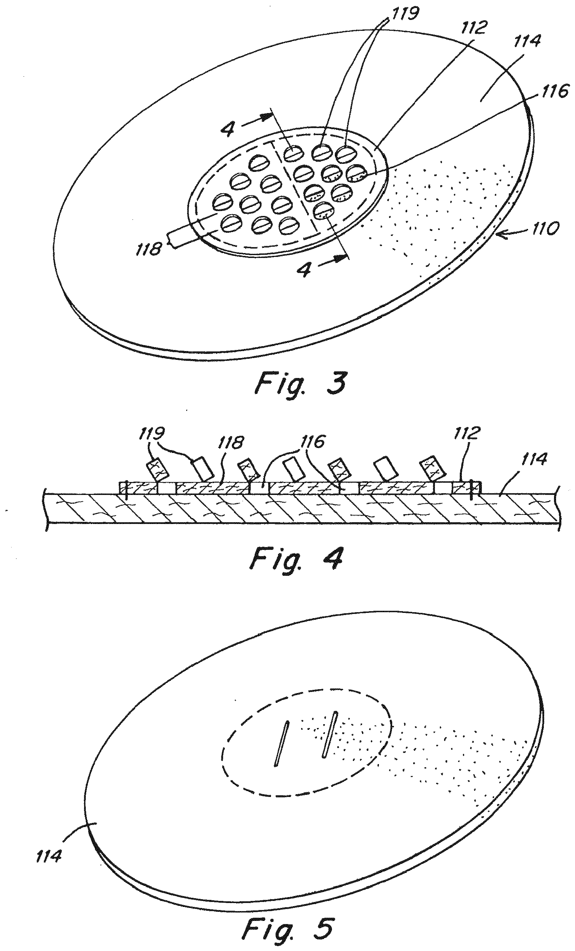

According to another embodiment of the invention, illustrated in FIGS. 3 through 5, a prosthetic implant 110 includes an ingrowth layer 112 formed of a porcine dermis material, which may be processed to be acellular and lyophilized or otherwise rendered suitable for implantation in a human or animal. Hinged tabs 119 are die cut or otherwise formed through the thickness of the ingrowth layer. The tabs 119 may then be folded or rotated out of the plane of the ingrowth layer forming openings 116 for tissue ingrowth. The tabs may be positioned to protrude into the plane of the tissue defect, providing an additional scaffold for ingrowth in the regions where the margins of the defect are not fully approximated. In the embodiment shown, tabs 119 have an arcuate or semi-circular profile. Advantageously, openings 116 provided in the ingrowth layer, encourage native tissue ingrowth and vascularization while reducing the potential for post-surgical seroma formation. It will be appreciated that the size, configuration, spacing and orientation of the tabs may be varied to meet particular needs. This prosthetic, and any of the other devices described herein, may additionally be impregnated, coated or otherwise configured with antibiotics, anesthetics, drugs, or other medicament; rendering, for example, a drug eluting prosthetic device.

### EXAMPLE

The following example is illustrative only and is not intended to limit the scope of the present invention. Burst strength characteristics were tested. Testing methodology and results appear below.

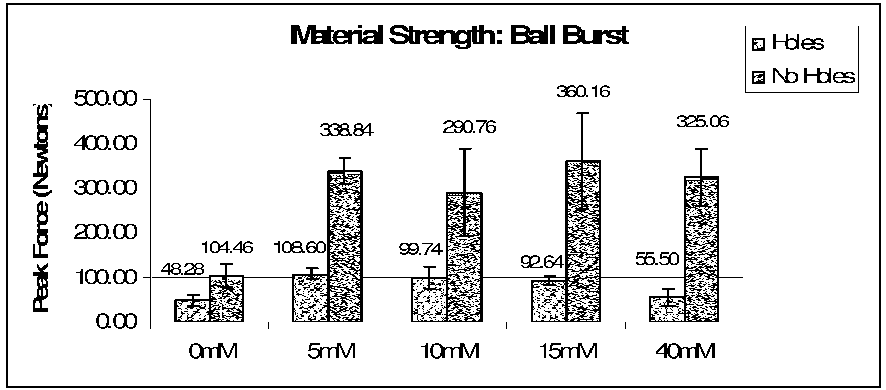

Ball burst strength was evaluated at five levels of crosslinking (0mM through 40 mM) of COLLAMEND [TM] material with and without holes. The results represent an average of at least 5 samples.

All samples were hydrated for 24 hours in 0.9% normal sterile saline prior to testing. All holes were 0.094-inches in diameter. A hole was formed in the center of the sample and five holes were located about the periphery at approximately 0.60 to 0.74-inches spacing. The samples were 1.5" circles. The ball burst testing followed, basically, the procedure recited in ASTM D3787-01, and utilized a 3/8-inch rod for bursting the samples in an Instron machine.

| Xlink | Holes | Holes st dev | No Holes | No Holes st dev |
|---|---|---|---|---|
| 0mM | 48.28 | 11.209 | 104.46 | 27.334 |
| 5mM | 108.60 | 13.185 | 338.84 | 29.852 |
| 10mM | 99.74 | 25.455 | 290.76 | 97.204 |
| 15mM | 92.64 | 11.095 | 360.16 | 107.930 |
| 40mM | 55.50 | 19.736 | 325.06 | 64.876 |

Although the invention has been described in terms of exemplary embodiments, it is not limited thereto. Rather, the appended claims should be construed broadly, to include other variants and embodiments of the invention, which may be made by those skilled in the art without departing from the scope and range of equivalents of the invention.

## Claims

1. A prosthetic device for repairing or augmenting a tissue, muscle or organ defect, comprising:
a first portion of biological tissue that is remodelable and configured for tissue ingrowth, said first portion constructed and arranged to cover or plug a defect; and
a second portion of biological tissue that is more slowly enzymatically degradable after implantation than the first layer and that is not configured for tissue ingrowth, said second portion having sufficient pullout strength so that said prosthetic device is fixatable through said second portion.

2. The prosthetic device of claim 1, wherein said first portion of biological tissue is not cross-linked or is minimally cross-linked.

3. The prosthetic device of claim 1, wherein said second portion of biological tissue is cross-linked.

4. The prosthetic device of claim 1, wherein at least 50% of said first portion of biological tissue is resorbable in less than twelve weeks after implantation.

5. The prosthetic device of claim 1, wherein at least 50% of said second portion of biological tissue is resorbable in no less than fifty-two weeks.

6. The prosthetic device of claim 1, wherein said first portion of biological tissue has a thickness of between about 0.3 mm and 0.5 mm.

7. The prosthetic device of claim 1, wherein said second portion of biological tissue has a thickness of between about 0.7 mm and 1.4 mm.

8. The prosthetic device of claim 1, wherein said first and second portions of biological tissue are acellular, lyophilized, porcine dermis.

9. The prosthetic device of claim 1, wherein said first and second portions of biological tissue are in the form of a patch.

10. The prosthetic device of claim 9, wherein said patch includes a sheet of said second portion of biological tissue that surrounds at least an aspect of a sheet of said first portion of biological tissue.

11. The prosthetic device of claim 10, wherein said sheet of said first portion of biological tissue is positioned against said sheet of said second portion of biological tissue.

12. The prosthetic device of claim 11, wherein said sheet of said first portion of biological tissue is positioned either face-to-face, or edge-to-edge, with said sheet of said second portion of biological tissue.

13. The prosthetic device of claim 12, wherein said sheet of said first portion of biological tissue is attached to said sheet of said second portion of biological tissue by a resorbable material.

14. The prosthetic device of claim 1, wherein said second portion of biological tissue has a ball burst strength at least about twice that of said first portion of biological tissue.

15. The prosthetic device of claim 14, wherein said second portion of biological tissue has a ball burst strength of about three times or at least about four times that of said first portion of biological tissue.
